(19) Europäisches Patentamt European Patent Office Office européen des brevets

(11) **EP 4 190 936 B1**

(12) **EUROPEAN PATENT SPECIFICATION**

(45) Date of publication and mention
of the grant of the patent:
**26.02.2025 Bulletin 2025/09**

(21) Application number: **21849148.8**

(22) Date of filing: **30.06.2021**

(51) International Patent Classification (IPC):
*C23C 4/04* (2006.01)    *C23C 4/134* (2016.01)
*A61L 27/04* (2006.01)    *A61L 27/06* (2006.01)
*A61L 27/10* (2006.01)    *A61L 27/18* (2006.01)
*A61L 27/32* (2006.01)    *A61L 27/50* (2006.01)
*A61L 27/56* (2006.01)    *A61F 2/30* (2006.01)

(52) Cooperative Patent Classification (CPC):
(C-Sets available)
**C23C 4/10; A61F 2/30767; A61L 27/32;
A61L 27/56; C04B 35/447; C04B 35/62222;
C04B 41/009; C04B 41/5048; C04B 41/87;
C23C 4/134;** A61F 2002/3092; A61F 2310/00796;
A61L 2430/02; C04B 2235/5436          (Cont.)

(86) International application number:
**PCT/JP2021/024830**

(87) International publication number:
**WO 2022/024652 (03.02.2022 Gazette 2022/05)**

(54) **PLASMA SPRAYING MATERIAL**

PLASMASPRITZMATERIAL

MATÉRIAU DE PROJECTION AU PLASMA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **30.07.2020   JP 2020129674**

(43) Date of publication of application:
**07.06.2023   Bulletin 2023/23**

(73) Proprietor: **Tomita Pharmaceutical Co., Ltd.
Naruto-shi, Tokushima 771-0360 (JP)**

(72) Inventors:
• **KITAMURA, Naoyuki
  Naruto-shi, Tokushima 771-0360 (JP)**
• **BANDO, Akihito
  Naruto-shi, Tokushima 771-0360 (JP)**
• **TSUMURA, Yuta
  Naruto-shi, Tokushima 771-0360 (JP)**
• **KUSHIKI, Yohei
  Naruto-shi, Tokushima 771-0360 (JP)**

(74) Representative: **Mewburn Ellis LLP
Aurora Building
Counterslip
Bristol BS1 6BX (GB)**

(56) References cited:
**WO-A1-01/81243      JP-A- 2003 325 553
JP-A- H1 072 666    JP-B1- 6 522 271**

• **BARILLAS: "Hydroxyapatite coatings on
  polymers using a custom low-energy plasma
  spray system", 1 January 2018 (2018-01-01),
  XP93184887**
• **BARILLAS: "Hydroxyapatite coatings on
  polymers using a custom low-energy plasma
  spray system", 1 January 2018 (2018-01-01),
  XP093184887, Retrieved from the Internet
  <URL:https://doi.org/10.1109/TPS.2018.2810639>**

- **BASTAN F E ET AL: "Spray drying of hydroxyapatite powders: The effect of spray drying parameters and heat treatment on the particle size and morphology", JOURNAL OF ALLOYS AND COMPOUNDS, ELSEVIER SEQUOIA, LAUSANNE, CH, vol. 724, 10 July 2017 (2017-07-10), pages 586 - 596, XP085149643, ISSN: 0925-8388, DOI: 10.1016/ J.JALLCOM.2017.07.116**
- **BASTAN, F. E. ET AL.: "Spray Braying of hydroxyapatite powders: The effect of spray drying parameters and heat treatment on the particle size and morphology", JOURNAL OF ALLOYS AND COMPOUNDS, vol. 724, 10 July 2017 (2017-07-10), pages 586 - 596, XP085149643, DOI: 10.1016/j.jallcom.2017.07.116**

(52) Cooperative Patent Classification (CPC): (Cont.)

C-Sets
**C04B 41/009, C04B 35/10;**
**C04B 41/009, C04B 35/48;**
**C04B 41/5048, C04B 41/4527**

**Description**

TECHNICAL FIELD

The present invention relates to a plasma spraying material that can be used, even under a condition of plasma spraying with low flame energy, for forming a hydroxyapatite film having high hardness and being less likely to abrade.

BACKGROUND ART

As a result of the aging of the population, cases are increasing in which an implant such as an artificial joint or a spinal fixation device is used mainly due to a bone fracture or coxarthrosis. As a substrate of an implant, a metal material, such as a titanium alloy or a Co-Cr-Ni alloy, having high strength and high stability has been conventionally often used. However, in spite of the high strength, such a metal material has a disadvantage that damage to an own bone is caused due to the low elasticity of the metal material, or a disadvantage that a bone lesion becomes difficult to diagnose due to the low radiolucency of the metal material.

Therefore, in order to solve the above-described disadvantage of the metal material, a resin material, such as a polyether ether ketone (hereinafter, sometimes referred to as a PEEK), having strength, elasticity, chemical resistance, and radiolucency similar to those of an own bone has been used as a substrate of an implant. However, in spite of the excellent physical properties, a PEEK has a problem that loosening of an implant occurs when a PEEK substrate is used because a PEEK has no bioactivity and does not directly bind to a biological tissue.

In order to solve the above-described problem of an implant having a PEEK substrate, for example, studies have been made on imparting biocompatibility (performance for directly binding to a biological tissue) with a method of coating a PEEK surface with a bioactive material or a method of kneading a PEEK and a bioactive material. However, in the kneading method, the original physical properties of a PEEK cannot be maintained because a bioactive material and the PEEK are kneaded, and furthermore, it is impossible to make the bioactive material present on the entire surface. Therefore, it is considered that a coating method is preferable in order to impart biocompatibility to the entire surface with maintaining the original physical properties of a PEEK.

Conventionally, hydroxyapatite (hereinafter, sometimes referred to as HAp) has been mainly used as a bioactive material, and as a method of coating with hydroxyapatite, film formation has been studied with a method such as a plasma spraying method, an immersion method, an electrophoresis method, or a flame spraying method, and the plasma spraying method is considered to be most preferable from the viewpoints of production efficiency and the penetration rate of equipment. A method of forming a film of HAp on a plastic material such as a PEEK with the plasma spraying method has been conventionally reported (see, for example, Patent Document 1).

Meanwhile, in the plasma spraying method, a voltage is applied between a cathode and an anode to generate an arc, the arc is supplied with a working gas such as an argon gas to ionize the working gas and generate a plasma flame, the plasma flame is supplied with a plasma spraying material, and thus the coating material melted at the temperature with the stream of the plasma flame adheres to a substrate to form a film. The temperature of the plasma flame reaches around 10000°C, and therefore the substrate becomes hot. In the case of a metal substrate, the substrate is not decomposed by flame heat, but in the case of a resin substrate, thermal decomposition or thermal alteration may be caused.

L.Barillas et al., IEEE Transactions on Plasma Science , vol.46, n°7, July 2018 "Hydroxyapatite Coatings on Polymers using a Custom Low-Energy Plasma Spray System" discloses the deposition of hydroxypatite with an average grain size of 5 microns using a custom low-energy plasma spray system. Poor mechanical adhesion was observed possibly due to the fact that the hydroxyapatite particles did not reach sufficient temperature to properly melt and adhere to the surface. To overcome this problem a $TiO_2$ layer was deposited first.

PRIOR ART DOCUMENT

PATENT DOCUMENT

Patent Document 1: Japanese Patent Laid-open Publication No. 4-146762

SUMMARY OF THE INVENTION

PROBLEMS TO BE SOLVED BY THE INVENTION

In the case of forming an HAp film on a resin substrate such as a PEEK substrate with the plasma spraying method, the flame energy during plasma spraying is to be reduced in order to inhibit the resin substrate from thermal decomposition and thermal alteration. However, if the flame energy is reduced, film formation may be impossible, or a formed film may have insufficient hardness and may be likely to abrade. Therefore, there is a problem that controlling the conditions of plasma spraying is insufficient to form an HAp film having high hardness on a resin substrate such as a PEEK substrate.

Therefore, an object of the present invention is to provide a plasma spraying material that can be used, even under a condition of plasma spraying with low flame energy, for forming an HAp film having high hardness and being less likely to abrade.

MEANS FOR SOLVING THE PROBLEM

As a result of intensive studies, the present inventors have found that an HAp powder having an average particle size ($D_{50}$) of 15 to 40 $\mu$m and, as measured by mercury porosimetry, a pore volume of 0.01 to 0.30 cc/g at a pore size of 2000 nm or less can be used for plasma spraying to form an HAp film having high hardness and being less likely to abrade even under a condition of plasma spraying with low flame energy, and that the HAp film can be formed without thermal decomposition and thermal alteration of a resin substrate. The present invention has been completed by further studies based on the above-described findings.

That is, the present invention provides the invention of the aspects described below.

Item 1. A plasma spraying material including an HAp powder having an average particle size ($D_{50}$) of 15 to 40 $\mu$m and, as measured by mercury porosimetry, a pore volume of 0.01 to 0.30 cc/g at a pore size of 2000 nm or less.

Item 2. The plasma spraying material according to Item 1, wherein the pore volume is 0.01 to 0.25 cc/g.

Item 3. The plasma spraying material according to Item 1 or 2, wherein the average particle size ($D_{50}$) is 20 to 40 $\mu$m.

Item 4. The plasma spraying material according to any one of Items 1 to 3, wherein the HAp powder has a BET specific surface area of less than 5 $m^2$/g.

Item 5. The plasma spraying material according to any one of Items 1 to 4, wherein the HAp powder has a pore volume of 0.20 to 0.80 cc/g at a pore size of 2000 nm or more as measured by mercury porosimetry.

Item 6. The plasma spraying material according to any one of Items 1 to 5, the plasma spraying material to be used for plasma spraying in which a gas consisting of one or more kinds of monatomic molecules as a working gas.

Item 7. The plasma spraying material according to any one of Items 1 to 6, the plasma spraying material to be used for film formation on a substrate.

Item 8. The plasma spraying material according to Item 7, wherein a material of the substrate is a resin, a metal, or a ceramic.

Item 9. The plasma spraying material according to Item 7 or 8, wherein the material of the substrate is a polyether ether ketone.

Item 10. The plasma spraying material according to Item 7 or 8, wherein the material of the substrate is a titanium alloy.

Item 11. The plasma spraying material according to any one of Items 7 to 10, wherein the substrate is an implant.

Item 12. A method for forming an HAp film, the method including plasma-spraying the plasma spraying material according to any one of Items 1 to 11 to form an HAp film on a substrate.

Item 13. The method for forming an HAp film according to Item 12, wherein a material of the substrate is a resin, a metal, or a ceramic.

Item 14. The method for forming an HAp film according to Item 12 or 13, wherein the material of the substrate is a polyether ether ketone.

Item 15. The method for forming an HAp film according to Item 12 or 13, wherein the material of the substrate is a titanium alloy.

Item 16. The method for forming an HAp film according to any one of Items 12 to 15, wherein the substrate is an implant.

Item 17. Use of an HAp powder as a plasma spraying material, the HAp powder having an average particle size ($D_{50}$) of 15 to 40 $\mu$m and, as measured by mercury porosimetry, a pore volume of 0.01 to 0.30 cc/g at a pore size of 2000 nm or less.

ADVANTAGES OF THE INVENTION

Using the plasma spraying material of the present invention, plasma spraying can be performed, even under a condition of plasma spraying with low flame energy, to form an HAp film having high hardness and being less likely to abrade. Therefore, by performing plasma spraying on a resin substrate such as a PEEK substrate using the plasma spraying material of the present invention with low flame energy, the resin substrate can be inhibited from thermal decomposition and thermal alteration.

Although the following description should not be limitedly interpreted, it is presumed that in the plasma spraying material of the present invention, the entire particles in the used HAp powder can be melted even with low flame energy because the average particle size is limited to a relatively small range, and that heat transfer to the inside of the particles is not blocked, the heat of the plasma flame is easily transferred to the entire particles, and the collision energy to the substrate is high because few voids are present in the particles resulting from that the pore volume at a pore size of 2000 nm or less measured by mercury porosimetry is limited to a relatively small range. It is considered that as a result of such characteristics, the plasma spraying material of the present invention can satisfy the required performance under a condition of plasma spraying with low flame energy.

BRIEF DESCRIPTION OF THE DRAWINGS

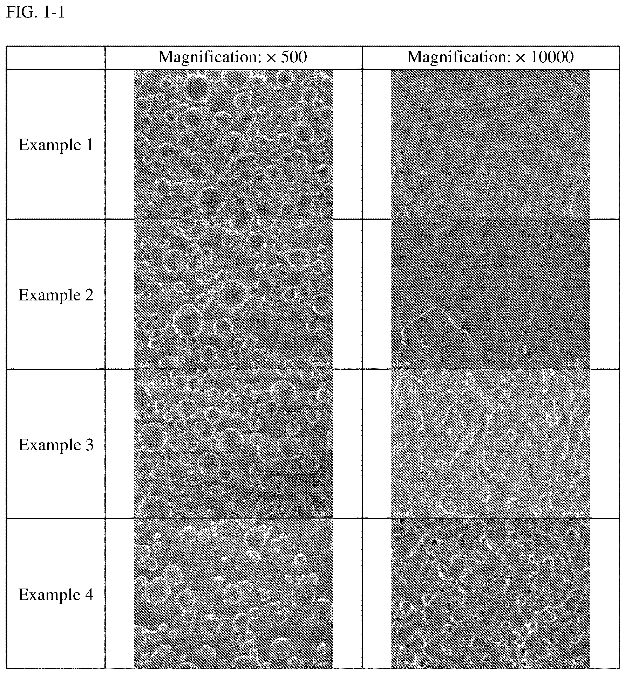

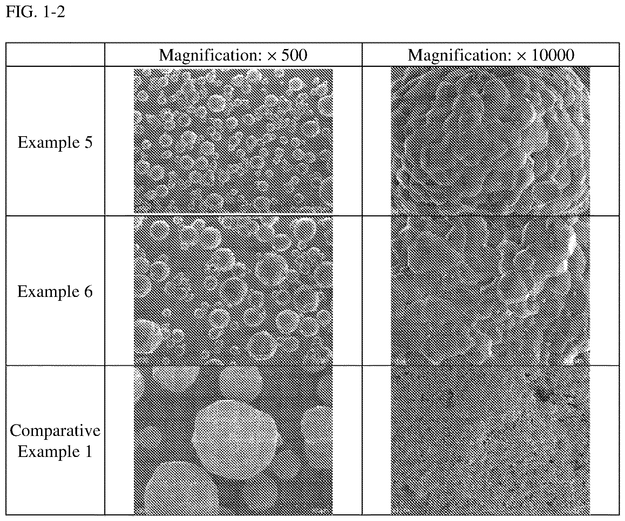

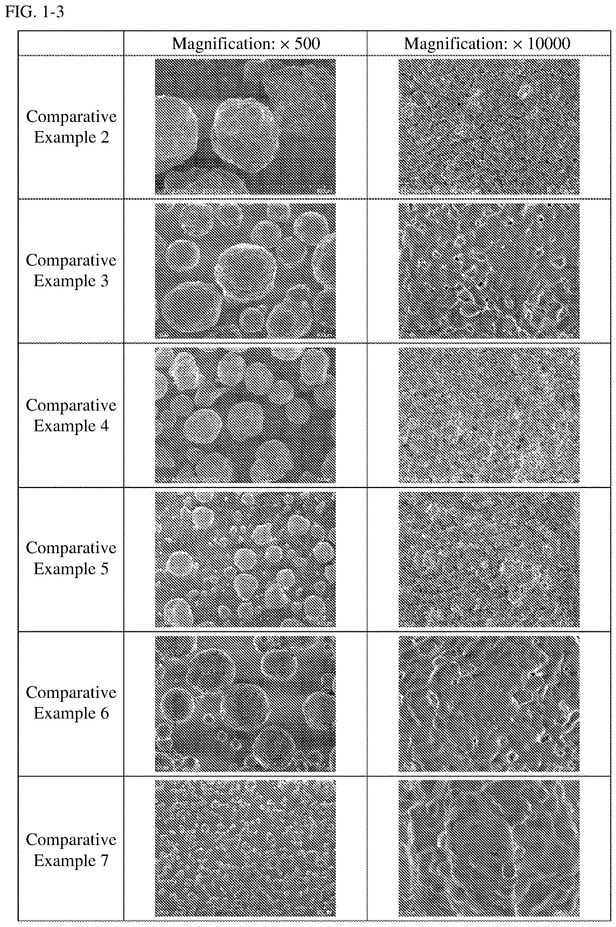

Fig. 1-1 shows images obtained by microscopically observing the appearance of HAp powders in Examples 1 to 4.
Fig. 1-2 shows images obtained by microscopically observing the appearance of HAp powders in Examples 5 to 6 and Comparative Example 1.
Fig. 1-3 shows images obtained by microscopically observing the appearance of HAp powders in Comparative Examples 2 to 7.
Fig. 2-1 shows images obtained by microscopically observing the cross-sectional appearance of HAp films formed by plasma spraying the HAp powders in Examples 1 to 4.
Fig. 2-2 shows images obtained by microscopically observing the cross-sectional appearance of HAp films formed by plasma spraying the HAp powders in Examples 5 to 6 and Comparative Example 5.
Fig. 3 shows results of determining the pore size distribution of the HAp powders in Examples 1 and 2 by mercury porosimetry.
Fig. 4 shows results of determining the pore size distribution of the HAp powders in Examples 3 and 4 by mercury porosimetry.
Fig. 5 shows results of determining the pore size distribution of the HAp powders in Comparative Examples 1 to 3 by mercury porosimetry.
Fig. 6 shows results of determining the pore size distribution of the HAp powders in Comparative Examples 4 and 5 by mercury porosimetry.
Fig. 7 shows results of determining the pore size distribution of the HAp powders in Comparative Examples 6 and 7 by mercury porosimetry.
Fig. 8 shows results of determining the pore size distribution of the HAp powders in Examples 5 and 6 by mercury porosimetry.
Fig. 9 shows a result of powder X-ray diffraction analysis of the HAp powder in Example 1.

EMBODIMENTS OF THE INVENTION

The plasma spraying material of the present invention includes an HAp powder having an average particle size ($D_{50}$) of 15 to 40 $\mu$m and, as measured by mercury porosimetry, a pore volume of 0.01 to 0.30 cc/g at a pore size of 2000 nm or less. Hereinafter, the plasma spraying material of the present invention will be described in detail.

[Physical Properties of HAp Powder]

HAp is calcium phosphate represented by the chemical formula $Ca_5(PO_4)_3(OH)$.
The HAp powder used in the present invention has an average particle size ($D_{50}$) of 15 to 40 $\mu$m. The average particle size ($D_{50}$) of the HAp powder used in the present invention is preferably 20 to 40 $\mu$m, more preferably 20 to 30 $\mu$m, still more preferably 25 to 30 $\mu$m, and particularly preferably 26 to 30 $\mu$m. In the present invention, the term "average particle size ($D_{50}$)" refers to the particle size (median diameter) at which the cumulative relative frequency is 50% in volume-based cumulative particle size distribution measured using a laser diffraction/scattering type particle size distribution measuring device.
The $D_{90}$ of the HAp powder used in the present invention is not particularly limited as long as the range of the average particle size ($D_{50}$) is satisfied, and the $D_{90}$ is, for example, 30 to 70 $\mu$m, preferably 35 to 55 $\mu$m, and more preferably 41 to 47 $\mu$m. In the present invention, the term "$D_{90}$" refers to the particle size at which the cumulative relative frequency is 90% in volume-based cumulative particle size distribution measured using a laser diffraction/scattering type particle size distribution measuring device.
The $D_{10}$ of the HAp powder used in the present invention is not particularly limited as long as the range of the average particle size ($D_{50}$) is satisfied, and the $D_{10}$ is, for example, 7 to 30 $\mu$m, preferably 10 to 25 $\mu$m, and more preferably 14 to 19 $\mu$m. In the present invention, the term "$D_{10}$" refers to the particle size at which the cumulative relative frequency is 10% in volume-based cumulative particle size distribution measured using a laser diffraction/scattering type particle size distribution measuring device.

The HAp powder used in the present invention has a pore volume of 0.01 to 0.30 cc/g at a pore size of 2000 nm or less as measured by mercury porosimetry. Satisfying the range of the average particle size and the range of the pore volume enables formation of a HAp film having high hardness and being less likely to abrade by plasma spraying even under a condition of plasma spraying with low flame energy. From the viewpoint of further improving the hardness and the abrasion resistance of the HAp film formed under a condition of plasma spraying with low flame energy, the pore volume at a pore size of 2000 nm or less measured by mercury porosimetry is preferably 0.01 to 0.25 cc/g, more preferably 0.01 to 0.23 cc/g, and still more preferably 0.01 to 0.22 cc/g.

In the present invention, the term "pore volume at a pore size of 2000 nm or less measured by mercury porosimetry" refers to the cumulative pore volume in a region in which the pore size is 2000 nm or less, in the pore volume measured using a mercury porosimeter. In the measurement of the pore volume, conditions of the mercury porosimeter are set such that a contact angle of mercury is 140° and a surface tension of mercury is 480 erg/cm$^2$.

In the HAp powder used in the present invention, the pore volume at a pore size of 2000 nm or more measured by mercury porosimetry is not particularly limited, and is, for example, 0.20 to 0.80 cc/g, preferably 0.30 to 0.75 cc/g, and more preferably 0.35 to 0.70 cc/g.

In the present invention, the term "pore volume at a pore size of 2000 nm or more measured by mercury porosimetry" refers to the cumulative pore volume in a region in which the pore size is 2000 nm or more, in the pore volume measured using a mercury porosimeter. In the measurement of the pore volume, conditions of the mercury porosimeter are the same as in the case of the "pore volume at a pore size of 2000 nm or less".

In the HAp powder used in the present invention, the mode diameter at a pore size of 2000 nm or less measured by mercury porosimetry is not particularly limited, and is, for example, 3 to 1000 nm, preferably 4 to 750 nm, and more preferably 5 to 500 nm.

In the present invention, the term "mode diameter at a pore size of 2000 nm or less measured by mercury porosimetry" refers to the diameter having the highest appearance rate (modal pore diameter) in a region in which the pore size is 2000 nm or less in the pore distribution measured using a mercury porosimeter. In the measurement of the mode diameter, conditions of the mercury porosimeter are the same as in the case of the "pore volume at a pore size of 2000 nm or less".

In the HAp powder used in the present invention, the mode diameter at a pore size of 2000 nm or more measured by mercury porosimetry is not particularly limited, and is, for example, 2000 to 15000 nm, preferably 5000 to 13000 nm, more preferably 7500 to 13000 nm, and particularly preferably 8000 to 11000 nm.

In the present invention, the term "mode diameter at a pore size of 2000 nm or more measured by mercury porosimetry" refers to the diameter having the highest appearance rate (modal pore diameter) in a region in which the pore size is 2000 nm or more in the pore distribution measured using a mercury porosimeter. In the measurement of the mode diameter, conditions of the mercury porosimeter are the same as in the case of the "pore volume at a pore size of 2000 nm or less".

In the HAp powder used in the present invention, the pore volume measured with a gas adsorption method is not particularly limited, and is, for example, 0.0001 to 0.01 cc/g, preferably 0.0005 to 0.005 cc/g, and more preferably 0.001 to 0.003 cc/g.

In the present invention, the term "pore volume measured with a gas adsorption method" refers to a value measured with the following method using a high-speed specific surface area/pore distribution measuring device. First, 1.0 to 2.0 g of the HAp powder is accurately weighed, enclosed in an adsorption tube, and degassed at 105°C for 3 hours. Next, the adsorption isotherm of a nitrogen gas is determined at the liquid nitrogen gas temperature, and the total pore volume (cc/g) is calculated from the gas adsorption amount at a liquid relative pressure $P/P_0$ ($P_0$: saturated vapor pressure) of 0.995.

In the HAp powder used in the present invention, the average pore size measured with a gas adsorption method is not particularly limited, and is, for example, 5 to 1000 nm, preferably 8 to 700 nm, and more preferably 10 to 550 nm.

In the present invention, the term "average pore size measured with a gas adsorption method" refers to a value calculated in accordance with the following formula.

$$\text{Average pore size (nm)} = 4V/S \times 1000$$

V: Pore volume (cc/g) measured with gas adsorption method

S: BET specific surface area (m$^2$/g)

The bulk density of the HAp powder used in the present invention is not particularly limited, and is, for example, 0.1 to 3.0 g/mL, preferably 0.4 to 2.0 g/mL, and more preferably 0.6 to 1.1 g/mL.

In the present invention, the term "bulk density" refers to an aerated bulk density measured in accordance with the test method specified in ASTM B212.

The BET specific surface area of the HAp powder used in the present invention is not particularly limited, and is, for example, less than 5 $m^2/g$, preferably 0 to 2 $m^2/g$, and more preferably 0 to 1 $m^2/g$.

In the present invention, the term "BET specific surface area" refers to a value measured with the following method using a high-speed specific surface area/pore distribution measuring device. First, 1.0 to 2.0 g of the HAp powder is accurately weighed, enclosed in an adsorption tube, and degassed at 105°C for 3 hours. Next, the adsorption isotherm of a nitrogen gas is determined at the liquid nitrogen gas temperature, and the specific surface area ($m^2/g$) is calculated with a multipoint BET method using the adsorption isotherm.

The angle of repose of the HAp powder used in the present invention is not particularly limited, and is, for example, 20 to 90°, preferably 40 to 70°, and more preferably 40 to 60°. In order to obtain an HAp powder having such an angle of repose, the particle shape of the HAp powder is preferably spherical, substantially spherical, or the like.

In the present invention, the term "angle of repose" refers to a value measured with an injection method at a vibration time set to 30 seconds and an amplitude set to 0.5 mm.

The particle hardness of the HAp powder used in the present invention is not particularly limited, and is, for example, 100 to 15000 $gf/mm^2$, preferably 500 to 10000 $gf/mm^2$, and more preferably 800 to 8000 $gf/mm^2$.

In the present invention, the term "particle hardness " of the HAp powder refers to a value determined by calculating the average of results of 10 times of measurement in which a particle hardness tester is used at a measurement speed set to 10 $\mu$m/s, a maximum value detection decreasing rate set to 80% (the threshold at which the peak value is read; when the load is decreased from the previous peak value by 20%, the previous peak value is detected as the maximum value), and a sample stage detection load (a load as the standard of detecting the origin position) set to 10.0 gf.

[Method for manufacturing HAp Powder]

The method for manufacturing the HAp powder used in the present invention is not particularly limited as long as an HAp powder having the above-described physical properties can be obtained, and preferred examples of the method include a method including the following first to fifth steps.

First step: HAp is produced with (1) a wet method including a sequential addition step of adding dropwise phosphoric acid to a suspension in which calcium hydroxide is suspended, or (2) a wet method including a sequential addition step of adding a suspension in which calcium hydroxide is suspended to a phosphoric acid aqueous solution in which phosphoric acid is dissolved in water.
Second step: The HAp obtained in the first step is wet-ground to obtain a wet-ground product of HAp.
Third step: The wet-ground product of HAp obtained in the second step is dried to obtain a dry HAp powder.
Fourth step: The dry HAp powder obtained in the third step is fired at a temperature higher than 1050°C and lower than 1400°C.
Fifth step: The fired HAp powder obtained in the fourth step is sieved to collect a HAp powder having an average particle size ($D_{50}$) of 15 to 40 $\mu$m.

In the first step, (1) phosphoric acid is added dropwise to a suspension in which calcium hydroxide is suspended, or (2) a suspension in which calcium hydroxide is suspended is added to a phosphoric acid aqueous solution in which phosphoric acid is dissolved in water to react calcium ions with phosphate ions, and thus a synthesis reaction of HAp [$10Ca(OH)_2 + 6H_3PO_4 \rightarrow Ca_{10}(PO_4)_6(OH)_2$] is to be carried out. In the first step, the ratio of calcium hydroxide and phosphoric acid finally coexistent with each other is to be adjusted to be similar to the ratio of calcium and phosphorus in HAp. A liquid in which calcium hydroxide is suspended in an emulsion form in water can be obtained by adding calcium oxide to water to cause a hydration reaction. In the case of adding dropwise phosphoric acid to a suspension in which calcium hydroxide is suspended, the phosphoric acid to be added dropwise is preferably in the state of a phosphoric acid aqueous solution in which phosphoric acid is dissolved in water. In the case of adding dropwise phosphoric acid to a suspension in which calcium hydroxide is suspended, the rate of adding dropwise phosphoric acid is to be appropriately adjusted so that the reaction solution after the dropwise addition has a pH of 9 or less, and the rate is in a range such that the rate of adding phosphorus (P) atoms is, for example, 0.05 to 0.6 mol/h, preferably 0.1 to 0.3 mol/h, and more preferably 0.2 mol/h based on 1 mol of calcium (Ca) atoms. In the case of adding a suspension in which calcium hydroxide is suspended to a phosphoric acid aqueous solution in which phosphoric acid is dissolved in water, the rate is in a range such that the rate of adding calcium (Ca) atoms is 0.05 to 0.6 mol/h based on 1 mol of phosphorus (P) atoms.

In the first step, the temperature at which calcium ions and phosphate ions are reacted (reaction temperature) are to be appropriately set according to the amount and the rate of dropwise addition and the like, and the temperature is in a range of, for example, 20°C or higher, preferably 30 to 70°C, and more preferably 40 to 60°C. In order to react calcium ions with phosphate ions to produce a reaction solution further efficiently, it is desirable that all of calcium ions and phosphate ions be allowed to coexist, and then subjected to aging under the above-described temperature condition. In the present invention, the term "aging" refers to leaving to stand or leaving under stirring for a certain period of time. The aging time is to be appropriately set according to the amount and the rate of dropwise addition, the reaction temperature, and the like, and is in a range of, for example, 0 minutes or more, preferably 0.5 to 5 hours, and more preferably 1 to 3 hours. Here, the term "aging time" refers to a time during which calcium ions and phosphate ions in water are left to stand or left under stirring from the time point at which all of the calcium ions and the phosphate ions coexist in water as 0 minute, and for example, refers to a time calculated, in the case of adding dropwise phosphoric acid to a suspension in which calcium hydroxide is suspended, when the end of dropwise addition of phosphoric acid is set to 0 minute.

If HAp is synthesized with a wet method in which a suspension in which calcium hydroxide is suspended and phosphoric acid are simultaneously mixed, the HAp cannot have the above-described physical properties, and HAp that can be subjected to plasma spraying cannot be formed.

From the viewpoint of efficiently manufacturing the HAp powder used in the present invention, the first step is preferably performed with a wet method including a sequential addition step of adding dropwise phosphoric acid to a suspension in which calcium hydroxide is suspended.

By performing the first step with such a method, a reaction solution in which HAp is produced is obtained.

In the second step, the HAp obtained in the first step is wet-ground to obtain a wet-ground product of HAp.

In the second step, the reaction solution after the first step as it is may be wet-ground, but a concentrated solution obtained by concentrating the reaction solution after the first step or a suspension obtained by collecting HAp from the reaction solution after the first step and suspending the HAp newly in water or an organic solvent such as an alcohol may be wet-ground.

In the second step, the method of wet-grinding is not particularly limited, and any wet-grinding may be performed such as impact grinding, shear grinding, grinding, compression grinding, or vibration grinding. The wet-grinder is not particularly limited, and may be any device such as a high-pressure fluid collision mill, a high-speed rotating slit mill, an attritor, a ball mill, a bead mill, a roll mill, a ring-shaped grinding medium mill, or a high-speed rotating thin film mill. As these devices, known or commercially available devices can be used. Among these wet-grinders, a bead mill can be suitably used.

In the case of using a bead mill as the wet-grinder, the beads are not particularly limited, but beads including a zirconia-based material are suitable. The size of the beads is to be, for example, about 0.1 to 3 mm in diameter. The filling amount of beads is to be appropriately set according to the size of the device to be used and the like, and for example, is to be appropriately adjusted within a range of about 50 to 90 volume%.

In the second step, the degree of wet-grinding is to be appropriately adjusted, and from the viewpoint of efficiently manufacturing an HAp powder having the above-described physical properties, the degree of wet-grinding is desirably adjusted so that in the HAp particles after wet-grinding, the average particle size is 10 $\mu$m or less and preferably 5 $\mu$m or less while the maximum particle size is 100 $\mu$m or less and preferably 30 $\mu$m or less, and the average particle size is more preferably 1 to 3 $\mu$m or less while the maximum particle size is 20 $\mu$m or less. In the present invention, the terms "average particle size" and "maximum particle size" of the HAp particles after wet-grinding refer to the median diameter (D50) and the maximum particle size that are measured using a laser diffraction/scattering type particle size distribution measuring device, respectively.

In the third step, the wet-ground product of HAp obtained in the second step is dried to obtain a dry HAp powder. The drying used in the third step is not particularly limited, and examples of the drying include spray drying, box type drying, band drying, vacuum drying, freeze drying, microwave drying, drum dryer, and fluidized drying.

Among them, spray drying is preferable from the viewpoint of obtaining spherical particles having a shape suitable for a plasma spraying material by drying the spherical particles. Spray drying conditions are not particularly limited as long as HAp having the above-described physical properties is obtained, and for example, the inlet temperature is to be set to 200 to 500°C, the outlet temperature is to be set to 100 to 200°C, and the disc rotation speed is to be set to 5000 to 30000 rpm.

In the fourth step, the dry HAp powder obtained in the third step is fired at a temperature higher than 1050°C and lower than 1400°C. Conventionally, an HAp powder manufactured with a wet method is generally fired under a temperature condition of 1000°C or lower, but under such a temperature condition, an HAp powder having the above-described physical properties cannot be obtained. In the fourth step, by setting the temperature condition of the firing treatment of the HAp powder manufactured in the third step to a temperature of higher than 1050°C and lower than 1400°C, an HAp powder having the above-described physical properties can be obtained. The temperature condition of the firing treatment in the fourth step is preferably higher than 1050°C to 1350°C, and more preferably 1100 to 1300°C.

The retention time of the temperature condition of the firing treatment in the fourth step is to be appropriately set in consideration of the temperature condition in a range in which an HAp powder having the above-described physical properties is produced. The above-described temperature condition of the firing treatment is to be reached even for a moment, and the retention time is preferably 0.1 to 10 hours, and more preferably 1 to 5 hours.

In the fifth step, the fired HAp powder obtained in the fourth step is sieved to collect an HAp powder having an

average particle size ($D_{50}$) of 15 to 40 $\mu$m. The mesh size of the sieve used in the fifth step is not particularly limited as long as an HAp powder having an average particle size ($D_{50}$) of 15 to 40 $\mu$m can be collected, and the mesh size is, for example, 500 $\mu$m or less, preferably 20 to 500 $\mu$m, and more preferably 30 to 50 $\mu$m.

By performing the fifth step with such a method, an HAp powder having the above-described physical properties (HAp powder to be used in the present invention) can be obtained.

The HAp powder to be used in the present invention can also be obtained by fractionating an HAp powder having the above-described physical properties by sieving or the like from an HAp powder manufactured with a method other than the manufacturing method including the first step to the fifth step.

[Application and Method of Use]

In the present invention, the HAp powder is used as a plasma spraying material. The term "plasma spraying material" refers to a powder to be subjected to plasma spraying (powder used as a raw material of a film to be formed). The term "plasma spraying" refers to a technique in which a plasma spraying material (powder) is heated by plasma and melted to form liquid fine particles, and the liquid fine particles are made to collide with a surface of a substrate at a high speed together with plasma jet to form a film of the plasma spraying material on the substrate.

In the plasma spraying in which the plasma spraying material of the present invention is used, the material of the substrate on which an HAp film is to be formed is not particularly limited, and examples of the material of the substrate include resins such as PEEKs, polyethylene, polyesters, polypropylene, polyamides, polyethers, polyether ketones, acrylic, polystyrene, polytetrafluoroethylene, hydroxyethyl methacrylate, polyamides, polylactic acid, polyglycolic acid, polylactide, polyglycolide, poly-para-dioxanone, trimethylene carbonate, and ε-caprolactone, metals such as titanium alloys (a Ti-6Al-4V alloy, Ni-Ti, and the like), cobalt alloys (a Co-Cr-Ni alloy, Co-Cr-Mo, Co-Cr-W-Ni, and the like), magnesium alloys (Mg-Y-RE, Mg-Ca-Zn, Mg-Li-Al, and the like), stainless steel (SUS 316L, SUS 304, and the like), titanium, cobalt, molybdenum, niobium, tantalum, gold, platinum, tungsten, iridium, and inconel, and ceramics such as alumina and zirconia. The plasma spraying material of the present invention can be subjected to plasma spraying even under a condition of plasma spraying with low flame energy to form an HAp film having high hardness and being less likely to abrade, and the resin substrate can be inhibited from thermal decomposition and thermal alteration by applying a condition of plasma spraying with low flame energy. In view of such an effect of the present invention, preferred examples of the material of the substrate on which an HAp film is to be formed include resins (particularly, PEEKs).

The kind (application) of the substrate on which an HAp film is to be formed is not particularly limited, and examples of the application include application to implants such as artificial joints, artificial dental roots, and artificial bones and housings of indwelling devices such as auxiliary artificial hearts, artificial blood vessels, stents, pacemakers, sutures, catheters, artificial skins, artificial muscles, and intraocular lenses. Among them, implants (particularly, artificial joints) are strongly required to be characterized in that the implants are likely to receive a load in a living body and that the HAp film provided on the substrate has high hardness and is less likely to abrade. The plasma spraying material of the present invention can form an HAp film that can satisfy the characteristics required for implants (particularly, artificial joints), and therefore the plasma spraying material is suitably used as a material for formation of an HAp film to be provided on a surface of an implant (particularly, an artificial joint).

The condition of plasma spraying when an HAp film is formed on a substrate using the plasma spraying material of the present invention is not particularly limited, and is to be appropriately set within the range of a condition of plasma spraying usually applied according to the kind of the substrate, the thickness of an HAp film to be formed, and the like.

As described above, the plasma spraying material of the present invention can be subjected to plasma spraying even under a condition of plasma spraying with low flame energy to form an HAp film having high hardness and being less likely to abrade, and therefore preferred examples of plasma spraying in which the plasma spraying material of the present invention is applied include a condition of plasma spraying with low flame energy. Specific examples of the condition of plasma spraying with low flame energy include conditions in which a gas including a monatomic molecule such as argon or helium at a high ratio is used as a working gas in plasma spraying. A monatomic molecule such as argon or helium has lower energy than a diatomic molecule such as hydrogen, nitrogen, or oxygen, and therefore by using a gas including a monatomic molecule at a high ratio as a working gas, the flame energy at the time of plasma spraying is reduced, and thus a plasma spraying condition can be obtained in which a resin substrate such as a PEEK substrate can be inhibited from thermal decomposition and thermal alteration. More specific examples of the working gas having low flame energy include gases consisting of one or more kinds of monatomic molecules only, and preferably a mixed gas of argon and helium.

Examples

Hereinafter, the present invention will be described more specifically with reference to Examples, but the present invention is not limited thereto.

1. Manufacture of Plasma Spraying Material (HAp Powder)

Example 1

Into a reaction tank, 6 L of water and 1 kg of calcium oxide were put to cause a hydration reaction, and then water was added to the suspension to adjust the total to 15 L. Next, the resulting mixture was heated to 50°C, and a phosphoric acid aqueous solution was added, until the pH reached 8, at a rate of dropwise addition such that the rate of adding phosphorus (P) atoms was 0.2 mol/h based on 1 mol of calcium (Ca) atoms. The obtained solution was heated to 95°C and reacted for 2 hours.

Next, the obtained reaction solution was wet-ground with zirconia beads having a diameter of 1 mm (bead filling amount: 70% (v/v)) until the average particle size reached 2 $\mu$m and the maximum particle size reached 15 $\mu$m to obtain a wet-ground product, and then the wet-ground product was spray-dried using a spray dryer equipped with a disk-type spraying means at an inlet temperature of 300°C, an outlet temperature of 130°C, and a disc rotation speed of 16000 rpm to collect a dry product.

Furthermore, the obtained dry product was fired using an electric furnace (manufactured by Kusaba Chemical) at 1220°C for 3 hours (temperature rising rate: 65°C/h). After cooling the fired product, the cooled product was sieved using a tabletop sieve shaker (VSS-200S manufactured by TSUTSUI SCIENTIFIC INSTRUMENTS CO., LTD.) under the condition A described in Table 1, and a powder passed through the sieve was collected to obtain an HAp powder.

Example 2

An HAp powder was obtained under the same conditions as in Example 1 except that the firing temperature was changed to 1300°C.

Example 3

An HAp powder was obtained under the same conditions as in Example 1 except that the firing temperature was changed to 1150°C.

Example 4

Into a reaction tank, 6 L of water and 1 kg of calcium oxide were put to cause a hydration reaction, and then water was added to the suspension to adjust the total to 15 L. Next, the resulting mixture was heated to 50°C, and a phosphoric acid aqueous solution was added, until the pH reached 8, at a rate of dropwise addition such that the rate of adding phosphorus (P) atoms was 0.2 mol/h based on 1 mol of calcium (Ca) atoms. The obtained solution was heated to 95°C and reacted for 2 hours.

Next, the obtained reaction solution was spray-dried using a spray dryer equipped with a disk-type spraying means at an inlet temperature of 300°C, an outlet temperature of 130°C, and a disc rotation speed of 10000 rpm to collect a dry product.

Furthermore, the obtained dry product was fired using an electric furnace (manufactured by Kusaba Chemical) at 1150°C for 3 hours (temperature rising rate: 65°C/h). After cooling the fired product, the cooled product was sieved using a tabletop sieve shaker (VSS-200S manufactured by TSUTSUI SCIENTIFIC INSTRUMENTS CO., LTD.) under the condition B described in Table 1, and a powder passed through the sieve was collected to obtain an HAp powder.

Example 5

The HAp powder obtained in Example 1 was sieved using a tabletop sieve shaker (VSS-200S manufactured by TSUTSUI SCIENTIFIC INSTRUMENTS CO., LTD.) under the condition C described in Table 1, and a powder passed through the sieve was collected to obtain an HAp powder.

Example 6

The HAp powder obtained in Example 1 was sieved using a tabletop sieve shaker (VSS-200S manufactured by TSUTSUI SCIENTIFIC INSTRUMENTS CO., LTD.) under the condition C described in Table 1, and a powder remaining on the sieve was collected to obtain an HAp powder.

Comparative Example 1

Into a reaction tank, 6 L of water and 1 kg of calcium oxide were put to cause a hydration reaction, and then water was added to the suspension to adjust the total to 15 L. Next, the resulting mixture was heated to 50°C, and a phosphoric acid aqueous solution was added, until the pH reached 8, at a rate of dropwise addition such that the rate of adding phosphorus (P) atoms was 0.2 mol/h based on 1 mol of calcium (Ca) atoms. The obtained solution was heated to 95°C and reacted for 2 hours.

Next, the obtained reaction solution was spray-dried using a spray dryer equipped with a disk-type spraying means at an inlet temperature of 300°C, an outlet temperature of 130°C, and a disc rotation speed of 11000 rpm to collect a dry product.

Furthermore, the obtained dry product was fired using an electric furnace (manufactured by Kusaba Chemical) at 800°C for 3 hours (temperature rising rate: 65°C/h). After cooling the fired product, an HAp powder was obtained.

Comparative Example 2

A commercially available HAp powder (Hydroxyapatite (Medipure20-15 No. 101) manufactured by Medicoat AG) was used.

Comparative Example 3

Into a reaction tank, 6 L of water and 1 kg of calcium oxide were put to cause a hydration reaction, and then water was added to the suspension to adjust the total to 15 L. Next, the resulting mixture was heated to 50°C, and a phosphoric acid aqueous solution was added, until the pH reached 8, at a rate of dropwise addition such that the rate of adding phosphorus (P) atoms was 0.2 mol/h based on 1 mol of calcium (Ca) atoms. The obtained solution was heated to 95°C and reacted for 2 hours.

Next, the obtained reaction solution was spray-dried using a spray dryer equipped with a disk-type spraying means at an inlet temperature of 300°C, an outlet temperature of 130°C, and a disc rotation speed of 10000 rpm to collect a dry product.

Furthermore, the obtained dry product was fired using an electric furnace (manufactured by Kusaba Chemical) at 1150°C for 3 hours (temperature rising rate: 65°C/h). After cooling the fired product, an HAp powder was obtained.

Comparative Example 4

A commercially available HAp powder (Hydroxyapatite (Medipure20-15 No. 102) manufactured by Medicoat AG) was used.

Comparative Example 5

Into a reaction tank, 6 L of water and 1 kg of calcium oxide were put to cause a hydration reaction, and then water was added to the suspension to adjust the total to 15 L. Next, the resulting mixture was heated to 50°C, and a phosphoric acid aqueous solution was added, until the pH reached 8, at a rate of dropwise addition such that the rate of adding phosphorus (P) atoms was 0.2 mol/h based on 1 mol of calcium (Ca) atoms. The obtained solution was heated to 95°C and reacted for 2 hours.

Next, the obtained reaction solution was spray-dried using a spray dryer equipped with a disk-type spraying means at an inlet temperature of 300°C, an outlet temperature of 130°C, and a disc rotation speed of 11000 rpm to collect a dry product.

Furthermore, the obtained dry product was fired using an electric furnace (manufactured by Kusaba Chemical) at 800°C for 3 hours (temperature rising rate: 65°C/h). After cooling the fired product, the cooled product was sieved using a tabletop sieve shaker (VSS-200S manufactured by TSUTSUI SCIENTIFIC INSTRUMENTS CO., LTD.) under the condition D described in Table 1, and a powder passed through the sieve was collected to obtain an HAp powder.

Comparative Example 6

Into a reaction tank, 6 L of water and 1 kg of calcium oxide were put to cause a hydration reaction, and then water was added to the suspension to adjust the total to 15 L. Next, the resulting mixture was heated to 50°C, and a phosphoric acid aqueous solution was added, until the pH reached 8, at a rate of dropwise addition such that the rate of adding phosphorus (P) atoms was 0.2 mol/h based on 1 mol of calcium (Ca) atoms. The obtained solution was heated to 95°C and reacted for 2 hours.

Next, the obtained reaction solution was wet-ground with zirconia beads having a diameter of 1 mm (bead filling amount: 70% (v/v)) until the average particle size reached 2 $\mu$m and the maximum particle size reached 15 $\mu$m to obtain a wet-ground product, and then the wet-ground product was spray-dried using a spray dryer equipped with a disk-type spraying means at an inlet temperature of 300°C, an outlet temperature of 130°C, and a disc rotation speed of 16000 rpm to collect a dry product.

Furthermore, the obtained dry product was fired using an electric furnace (manufactured by Kusaba Chemical) at 1180°C for 3 hours (temperature rising rate: 65°C/h). After cooling the fired product, the cooled product was sieved using a tabletop sieve shaker (VSS-200S manufactured by TSUTSUI SCIENTIFIC INSTRUMENTS CO., LTD.) under the condition D described in Table 1, and a powder on the sieve was collected to obtain an HAp powder.

Comparative Example 7

To 5 L of water, 2.5 L of a 25 wt% calcium hydroxide suspension and 1 L of a 50 wt% phosphoric acid solution were simultaneously added dropwise at a pH of 7 over 3 hours.

Next, the obtained reaction solution was spray-dried using a spray dryer equipped with a disk-type spraying means at an inlet temperature of 300°C, an outlet temperature of 130°C, and a disc rotation speed of 10000 rpm to collect a dry product.

Furthermore, the obtained dry product was fired using an electric furnace (manufactured by Kusaba Chemical) at 1200°C for 3 hours (temperature rising rate: 65°C/h). After cooling the fired product, an HAp powder was obtained.

Sieving Condition

Table 1 shows the sieving conditions applied to manufacture of the HAp powder in Examples 1 to 6 and Comparative Examples 5 to 6 described above.

[Table 1]

| | Condition A | Condition B | Condition C | Condition D |
|---|---|---|---|---|
| Mesh size of sieve | 45 $\mu$m | 45 $\mu$m | 45 $\mu$m | 45 $\mu$m |
| Number of sieves[#1] | 1 | 1 | 2 | 1 |
| Amount of charge | 50 g | 50 g | 100 g | 50 g |
| Vibration time | 180 sec | 180 sec | 10 sec | 180 sec |
| Vibration intensity[#2] | 5 | 5 | 5 | 5 |
| Sieve size | 20 cm | 20 cm | 20 cm | 20 cm |
| Number of times of sieving[#3] | 3 | 10 | 1 | 3 |

#1 The term "number of sieves" refers to the number of used sieves. For example, in the condition A, one sieve having a mesh size of 45 $\mu$m was used. In the condition C, two sieves having a mesh size of 45 $\mu$m were stacked and used, and powders remaining on the two sieves were collected and mixed to obtain an Hap powder.
#2 The term "vibration intensity" refers to a vibration intensity set in a tabletop sieve shaker (VSS-200S manufactured by TSUTSUI SCIENTIFIC INSTRUMENTS CO., LTD.).
#3 The term "number of times of sieving" refers to the times of sieving under each condition. For example, in the condition A, after 180 seconds of vibration time, sieving was repeated two times under the same condition to perform sieving total 3 times (for a total vibration time of 540 seconds), and the number of times of sieving is 3. When sieving was repeated, replenishment of the powder and return of the powder passed through the sieve were not performed.

2. Method of Evaluating Plasma Spraying Material

2-1. Evaluation of Physical Properties of Plasma Spraying Material

The HAp powders of Examples 1 to 6 and Comparative Examples 1 to 7 are each subjected to evaluation of the bulk density, the particle size distribution, the mode diameter and the pore volume at a pore size of 2000 nm or less/2000 nm or more (mercury porosimetry), the BET specific surface area, the pore volume (gas adsorption method), the average pore size (gas adsorption method), the crystallinity, the angle of repose, the particle hardness, and the appearance with the following methods.

Bulk Density

The bulk density (aerated bulk density) was measured in accordance with the test method specified in ASTM B212.

Particle Size Distribution

The HAp powder was dispersed in water, and the particle size distribution was measured using a laser diffraction/scattering type particle size distribution measuring device ("MICROTRAC MT3300 EXII" manufactured by MicrotracBEL Corp.) to determine D10, D50 (average particle size), and D90.

Mode Diameter and Pore Volume (Mercury Porosimetry) at Pore Size of 2000 nm or Less/2000 nm or More

The mode diameter and the pore volume were measured using a mercury porosimeter ("poremaster 60GT" manufactured by Quantachrome Corporation) under the following conditions. In a measurement cell, 0.1 to 1.0 g of the HAp powder was enclosed, the contact angle of mercury was set to 140° and the surface tension of mercury was set to 480 erg/cm$^2$, and the mode diameter and the pore volume were calculated from the measured pressure. The analysis range was divided into a range of a pore size of 2000 nm or less and a range of a pore size of 2000 nm or more.

BET Specific Surface Area

The BET specific surface area was measured using a high-speed specific surface area/pore distribution measuring device ("NOVA-4000" manufactured by Quantachrome Corporation) under the following operating conditions. Pretreatment: 1.0 to 2.0 g of the HAp powder was accurately weighed, enclosed in an adsorption tube, and degassed at 105°C for 3 hours.
Measurement and analysis: The adsorption isotherm of a nitrogen gas was determined at the liquid nitrogen gas temperature, and the specific surface area (m$^2$/g) was calculated with a multipoint BET method using the adsorption isotherm.

Pore Volume (Gas Adsorption Method)

The pore volume was measured with a gas adsorption method using a high-speed specific surface area/pore distribution measuring device ("NOVA-4000" manufactured by Quantachrome Corporation) under the following operating conditions. Pretreatment: 1.0 to 2.0 g of the HAp powder was accurately weighed, enclosed in an adsorption tube, and degassed at 105°C for 3 hours.
Measurement and analysis: The adsorption isotherm of a nitrogen gas was determined at the liquid nitrogen gas temperature, and the total pore volume (cc/g) was calculated from the gas adsorption amount at a relative pressure $P/P_0$ ($P_0$: saturated vapor pressure) of 0.995.

Average Pore Size (Gas Adsorption Method)

The average pore size (gas adsorption method) was calculated with the following formula.

$$\text{Average pore size (nm)} = 4V/S \times 1000$$

V: Pore volume (gas adsorption method) (cc/g)
S: BET specific surface area (m$^2$/g)

Crystallinity

The diffraction pattern of the HAp powder (test specimen) and the diffraction pattern of the sample obtained by treating the HAp powder at 1000°C for 15 hours (pretreatment product) were measured with an X-ray diffractometer "SmartLab" (manufactured by Rigaku Corporation) in the range of 2θ = 25 to 50° (measurement conditions: target: Cu, tube voltage: 40 kV, tube current: 30 mA, scanning range: 25 to 50°, scanning speed: 1.000°/min, scanning step: 0.02°, scanning mode: continuous). The diffraction angle (θ) corresponding to each lattice spacing (d) shown in Table 2 below was calculated from Bragg's equation d = λ/2sinθ, and for diffraction peaks having a peak top at the diffraction angle (2θ), the ratio of the sum of the integrated intensities of the peaks of the test specimen to the sum of the integrated intensities of the peaks of the pretreatment product was calculated.

[Table 2]

| Lattice spacing (d) |
| --- |
| d1 = 3.44 × 10$^{-10}$ m |
| d2 = 3.17 × 10$^{-10}$ m |
| d3 = 3.08 × 10$^{-10}$ m |
| d4 = 2.81 × 10$^{-10}$ m |
| d5 = 2.78 × 10$^{-10}$ m |
| d6 = 2.72 × 10$^{-10}$ m |
| d7 = 2.63 × 10$^{-10}$ m |
| d8 = 2.26 × 10$^{-10}$ m |
| d9 = 1.94 × 10$^{-10}$ m |
| d10 = 1.84 × 10$^{-10}$ m |

Angle of Repose

The angle of repose was measured using POWDER TESTER PT-X (manufactured by HOSOKAWA MICRON CORPORATION) at a vibration time set to 30 seconds, an amplitude set to 0.5 mm, and a frequency set to 50 Hz.

Particle Hardness

The particle hardness of the HAp powder was measured using a particle hardness measuring device New GRANO GM-N (manufactured by OKADA SEIKO CO., LTD.) under the following conditions.
Measurement conditions: The measurement was performed 10 times at a measurement speed set to 10 $\mu$m/s, a maximum value detection decreasing rate set to 80%, and a sample stage detection load set to 10.0 gf, and the average of the measurement results was calculated.

Appearance

The appearance of each HAp powder was observed using a field-emission scanning electron microscope at a magnification of 500 and 10000.

Powder X-ray Diffraction Analysis

Measurement was performed with an X-ray diffractometer "SmartLab" (manufactured by Rigaku Corporation) in the range of $2\theta$ = 25 to 50° (measurement conditions: target: Cu, tube voltage: 40 kV, tube current: 30 mA, scanning range: 20 to 50°, scanning speed: 1.000°/min, scanning step: 0.02°, scanning mode: continuous).

2-2. Plasma spray test

A surface of a Ti-6Al-4V alloy substrate (length: 30 mm, width: 40 mm, thickness: 3 mm) was roughened by blast treatment, and then an HAp film was formed using the HAp powder in each of Examples 1, 5 and 6 under the plasma spraying condition shown in Table 3 under atmospheric pressure. Furthermore, a surface of a PEEK substrate (length: 30 mm, width: 40 mm, thickness: 5 mm) was roughened by blast treatment, and then an HAp film was formed using the HAp powder in each of Examples 1 to 4 and Comparative Examples 1 to 7 under the plasma spraying condition shown in Table 3 under atmospheric pressure.
In the plasma spraying condition shown in Table 3 below, an argon gas and a helium gas are used as working gases, so that the flame energy is low. Therefore, even when plasma spraying was performed using HAp on the PEEK substrate, thermal deformation or thermal decomposition of the substrate did not occur.

[Table 3]

| Plasma spraying condition | | | |
|---|---|---|---|
| Item | | | Condition |
| Device | | | MF-P-HVOF-K1000 manufactured by GTV Verschleiss-Schutz Gmbh |
| Working gas | Ar | SLPM | 30 |
| | $H_e$ | SLPM | 150 |
| Carrier gas | $N_2$ | SLPM | 4 |
| Current value | | A | 500 |
| Spray distance | | mm | 100 |

Each formed HAp film was subjected to evaluation of the cross-sectional hardness, the film thickness, the surface roughness, the abrasion amount, the impurity phase ($\alpha$-TCP ($\alpha$-tricalcium phosphate), $\beta$-TCP ($\beta$-tricalcium phosphate), TTCP (tetracalcium phosphate), CaO), the color difference, the crystallinity, the Ca/P ratio, and the appearance with the following methods.

Cross-Sectional Hardness

The cross-sectional hardness of the HAp film at a test force of 0.3 kg was measured using a Vickers hardness tester.

Film Thickness

The thickness of the HAp film was measured using a micrometer.

Surface Roughness

The surface roughness (arithmetic average roughness: Ra) of the HAp film was measured using a surface roughness meter in accordance with JIS B 0031: 1994.

Abrasion Amount

The abrasion amount was measured using a Suga abrasion tester under a load of 1 N with abrasion paper SiC #320 after 100 times of abrasion.

Impurity Phase

A standard sample was prepared by mixing impurity phases with the HAp powder (Example 3) so that each impurity phase is contained at a predetermined content (0.5 wt%, 1.0 wt%, 2.5 wt%, 5.0 wt%, 6.0 wt%). Next, the diffraction pattern of the standard sample was measured with a powder X-ray diffractometer "SmartLab" (manufactured by Rigaku Corporation) in the range of $2\theta = 25$ to $50°$ (measurement conditions: target: Cu, tube voltage: 40 kV, tube current: 30 mA, scanning range: 25 to 50°, scanning speed: 1.000°/min, scanning step: 0.02°, scanning mode: continuous). The diffraction angle ($\theta$) corresponding to each lattice spacing (d) shown in Table 4 below was calculated from Bragg's equation $d = \lambda/2\sin\theta$, and the integrated intensity of the diffraction peak having a peak top at the diffraction angle ($2\theta$) was calculated. A calibration curve was prepared from the ratio of the integrated intensity of each impurity phase to the calculated integrated intensity of the HAp and the corresponding content of each impurity phase. Subsequently, the HAp film (test specimen) was measured under the same conditions, and the impurity phases were calculated from the HAp, the integrated intensity of each impurity phase, and the prepared calibration curve.

[Table 4]

| Crystal phase | Lattice spacing (d) |
|---|---|
| HAp | $3.08 \times 10^{-10}$ m |

(continued)

| Crystal phase | | Lattice spacing (d) |
|---|---|---|
| Impurity phase | α-TCP | $2.905 \times 10^{-10}$ m |
| | β-TCP | $2.88 \times 10^{-10}$ m |
| | TTCP | $2.995 \times 10^{-10}$ m |
| | CaO | $2.405 \times 10^{-10}$ m |

Ca/P Ratio

The Ca/P ratio of the HAp film was calculated from the contents of the impurity phases in accordance with the following formula.

[Math. 1]

$$Ca/P = nCa/nP$$
$$nCa = 10/M_{HA} \times W_{HA} + 3/M_{TCP} \times (W_{TCP\alpha} + W_{TCP\beta}) + 1/M_{CaO} \times W_{CaO}$$
$$nP = 6/M_{HA} \times W_{HA} + 2/M_{TCP} \times (W_{TCP\alpha} + W_{TCP\beta})$$

nCa: Number of moles of Ca

nP: Number of moles of P

$M_{HA}$: Molar mass of HAp

$M_{TCP}$: Molar mass of TCP

$M_{CaO}$: Molar mass of CaO

$W_{HA}$: Content of HAp

$W_{TCP\alpha}$: Content of α-TCP

$W_{TCP\beta}$: Content of β-TCP

$W_{CaO}$: Content of CaO

Crystallinity

The diffraction pattern of the HAp film (test specimen) and the diffraction pattern of the sample obtained by treating the HAp powder (Example 4) at 1000°C for 15 hours (pretreatment product) were measured with an X-ray diffractometer "SmartLab" (manufactured by Rigaku Corporation) in the range of $2\theta = 25$ to $50°$ (measurement conditions: target: Cu, tube voltage: 40 kV, tube current: 30 mA, scanning range: 25 to 50°, scanning speed: 24.000°/min, scanning step: 0.02°, scanning mode: continuous). The diffraction angle ($\theta$) corresponding to each lattice spacing (d) shown in Table 2 above was calculated from Bragg's equation $d = \lambda/2\sin\theta$, and for diffraction peaks having a peak top at the diffraction angle ($2\theta$), the ratio of the sum of the integrated intensities of the peaks of the test specimen to the sum of the integrated intensities of the peaks of the pretreatment product was calculated.

Color Difference

The L value, the a value, and the b value of the HAp film formed on the substrate were determined using a colorimetric color difference meter ("ZE 6000" manufactured by NIPPON DENSHOKU INDUSTRIES CO., LTD.) under a reflection condition, and the whiteness (W) was calculated in accordance with the following formula.

$$W = 100 - [(100 - L)^2 + (a^2 + b^2)]^{1/2}$$

Appearance

The cross section of the HAp film was observed using a scanning electron microscope at a magnification of 500 and 1000.

3. Evaluation Results

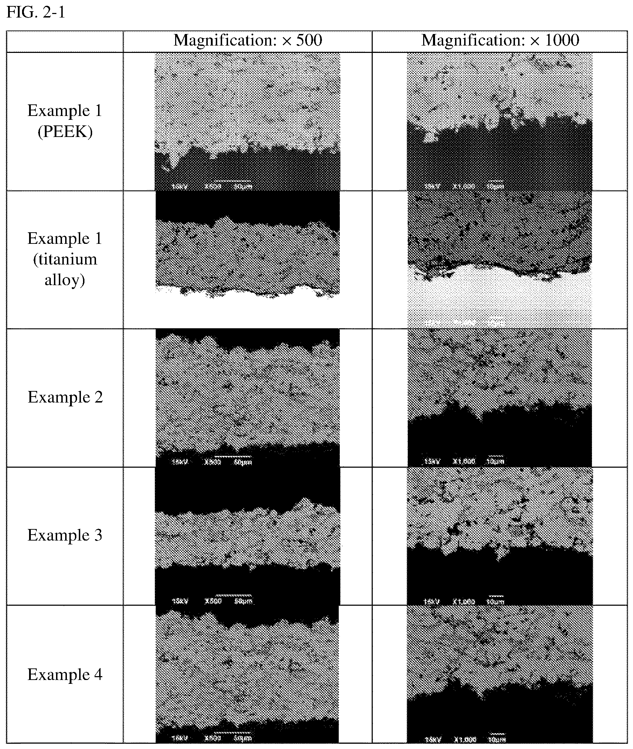

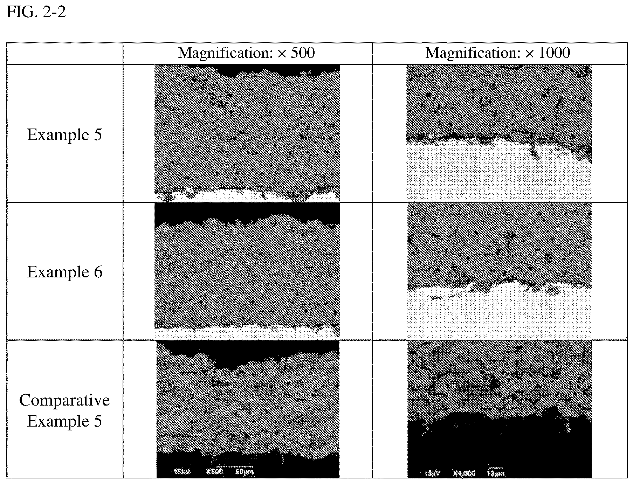

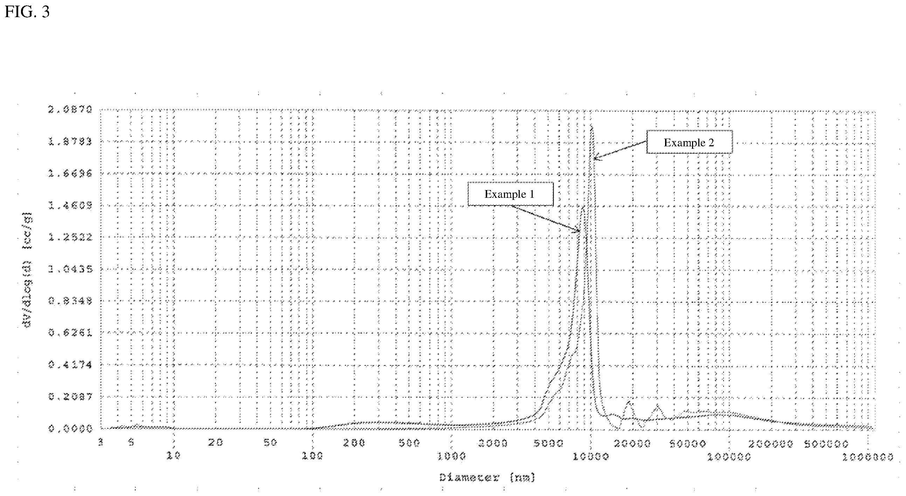

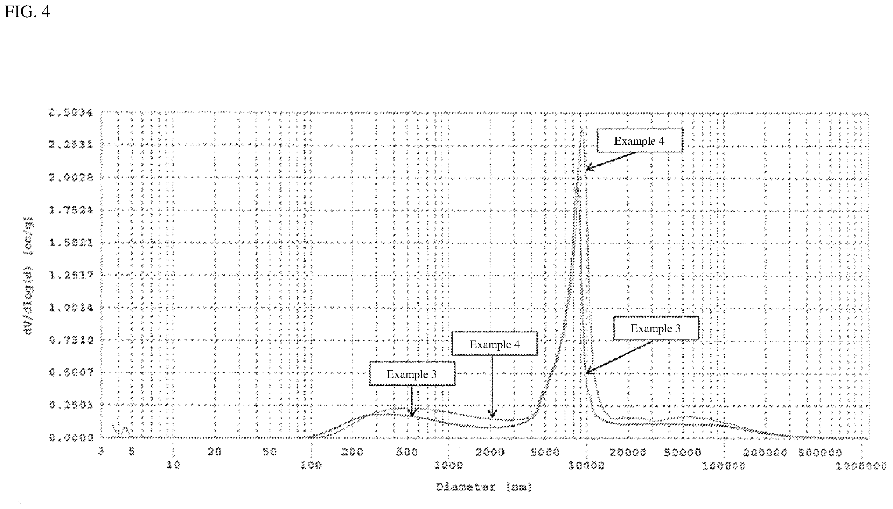

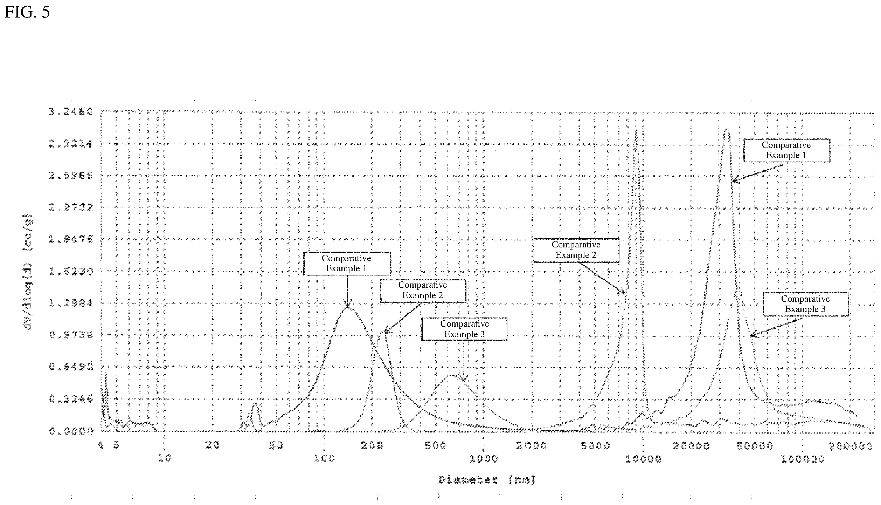

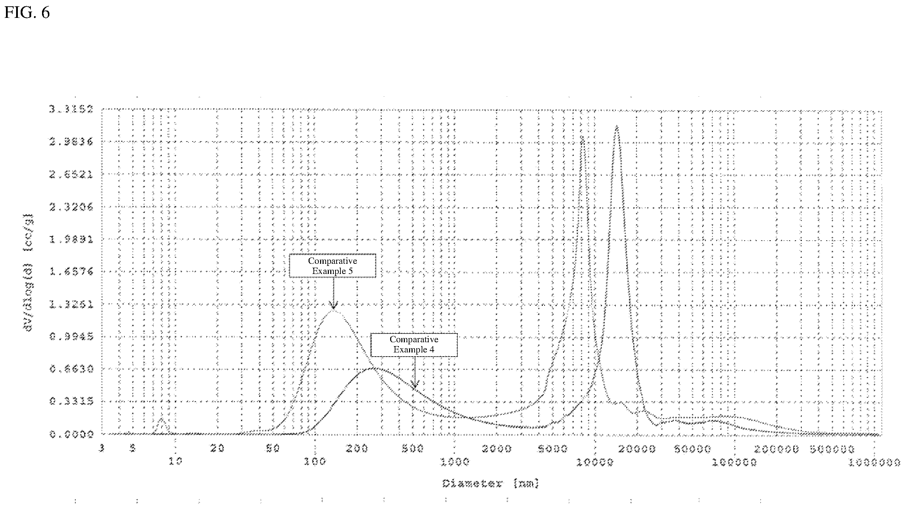

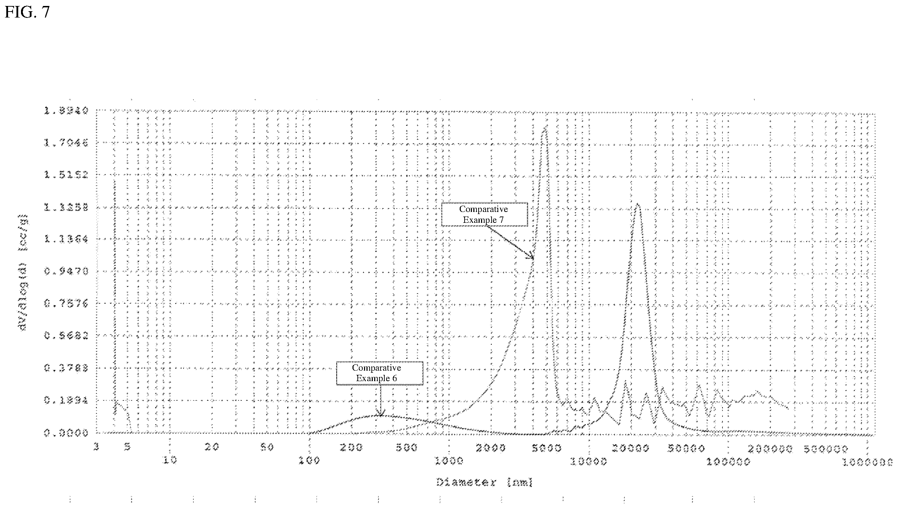

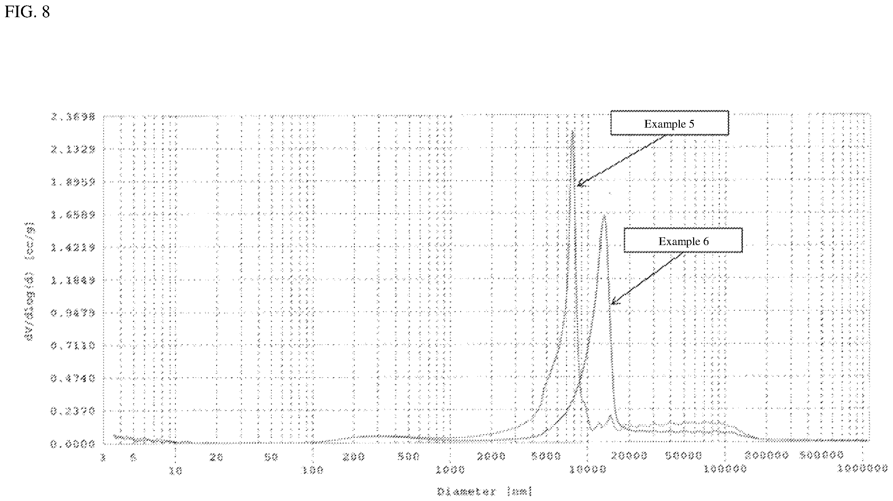

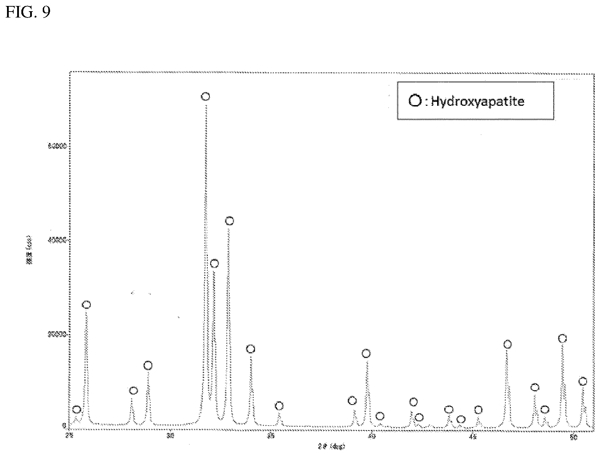

The obtained results are shown in Tables 5 and 6 and Figs. 1 to 9. Figs. 1-1, 1-2, and 1-3 show images obtained by microscopically observing the appearance of the HAp powders, Figs. 2-1 and 2-2 show images obtained by microscopically observing the cross-sectional appearance of the HAp films, Figs. 3 to 8 show results of measuring the pore size distribution of the HAp powders by mercury porosimetry, and Fig. 9 shows a result of powder X-ray diffraction analysis of the HAp powder in Example 1.

It is considered that the HAp powders of Comparative Examples 1 to 4 and Comparative Example 6 had such a large particle size that the energy of the plasma flame was insufficient to melt the particles themselves and form a film. It is considered that the HAp powder of Comparative Example 7 had such a low fluidity because of its small particle size that the HAp powder was not stably supplied to the thermal spraying device and as a result, no film was formed. It is considered that although the HAp powder of Comparative Example 5 had an appropriate particle size, the pore volume was so large that the heat of the plasma flame was not conducted to the entire particles and as a result, the unmelted powder remained in the film, the abrasion of the film was increased, and the hardness of the film was decreased.

Meanwhile, it has been confirmed that the HAp film formed with the plasma spraying method using the HAp powder in Examples 1 to 6 had a cross-sectional hardness of about 1.2 or more times that in Comparative Example 5 and a resistance concerning to the abrasion amount of 3.0 or more times that in Comparative Example 5. It is considered that the HAp powders of Examples 1 to 6 had an average particle size (D50) as small as 15 to 40 $\mu$m and a pore volume as small as 0.01 to 0.30 cc/g at a pore size of 2000 nm or less and therefore uniform melt of the particles was possible even with low flame energy and as a result, an HAp film having high hardness and a low abrasion amount was formed without causing thermal deformation or thermal decomposition not only in the titanium alloy substrate but also in the PEEK substrate. Furthermore, the HAp films formed using the HAp powders of Examples 1 to 6 also maintained a high crystallinity and a low impurity phase, and therefore suitable use of the HAp films as an implant was possible.

[Table 5]

| | Evaluation item | | Example 1 | | Example 2 | Example 3 | Example 4 | Example 5 | Example 6 |
|---|---|---|---|---|---|---|---|---|---|
| HAp powder | Bulk density (g/mL) | | 1.0 | | 1.0 | 0.8 | 0.6 | 0.9 | 1.1 |
| | Average particle size ($D_{50}$) ($\mu$m) | | 27 | | 29 | 29 | 29 | 20 | 36 |
| | Particle size distribution | $D_{10}$ ($\mu$m) | 16 | | 16 | 18 | 18 | 12 | 23 |
| | | $D_{50}$ ($\mu$m) | 27 | | 29 | 29 | 29 | 20 | 36 |
| | | $D_{90}$ ($\mu$m) | 44 | | 46 | 46 | 46 | 34 | 55 |
| | Mode diameter at pore size of 2000 nm or less (mercury porosimetry) (nm) | | 311 | | 6 | 325 | 496 | 4 | 4 |
| | Mode diameter at pore size of 2000 nm or more (mercury porosimetry) (nm) | | 8702 | | 10230 | 8507 | 9336 | 7664 | 12980 |
| | Pore volume at pore size of 2000 nm or less (mercury porosimetry) (cc/g) | | 0.04 | | 0.01 | 0.16 | 0.21 | 0.06 | 0.05 |
| | Pore volume at pore size of 2000 nm or more (mercury porosimetry) (cc/g) | | 0.39 | | 0.41 | 0.49 | 0.66 | 0.46 | 0.38 |
| | BET specific surface area (m$^2$/g) | | 0.5 | | 0.0 | 1.0 | 0.7 | 0.3 | 0.6 |
| | Average pore size (gas adsorption method) (nm) | | 11.5 | | 548 | 10.1 | 16.6 | 10.6 | 10.9 |
| | Pore volume (gas adsorption method) (cc/g) | | 0.002 | | 0.001 | 0.003 | 0.003 | 0.001 | 0.002 |
| | Crystallinity (%) | | 101 | | 99 | 99 | 98 | 99 | 100 |
| | Angle of repose (°) | | 52.6 | | 51.3 | 55.8 | 57.9 | 47.8 | 55.0 |
| | Particle hardness (gf/mm$^2$) | | 3897 | | 7624 | 853 | 907 | 3463 | 4958 |
| HAp film | Material of substrate | | Titanium alloy | PEEK | PEEK | PEEK | PEEK | Titanium alloy | Titanium alloy |
| | Cross-sectional hardness (HV0. 3) | | 200 | 244 | 199 | 173 | 182 | 236 | 222 |
| | Abrasion amount (mg) | | 6.7 | 4.2 | 4.7 | 29 | 27 | 7.5 | 13 |
| | Film thickness ($\mu$m) | | 110 | 170 | 120 | 110 | 140 | 160 | 140 |
| | Surface roughness (Ra, $\mu$m) | | 4.3 | 4.1 | 4.4 | 5.3 | 5.3 | 4.0 | 4.8 |
| | Crystallinity (%) | | 62 | 63 | 60 | 74 | 74 | 66 | 70 |
| | Impurity phase | $\alpha$-TCP | 0.3 | 1.4 | 1.0 | 0.6 | 1.5 | 0.6 | 0.4 |
| | | $\beta$-TCP | 0.7 | 1.6 | 0.8 | 1.3 | 1.2 | 0.5 | 0.7 |
| | | TTCP | 0.2 | 1.0 | 0.9 | 0.5 | 0.0 | 0.8 | 0.9 |
| | | CaO | 0.4 | 0.2 | 0.3 | 0.1 | 0.1 | 0.2 | 0.2 |
| | Ca/P ratio | | 1.68 | 1.67 | 1.67 | 1.67 | 1.67 | 1.67 | 1.67 |
| | Color difference | L | 85.5 | 87.5 | 85.0 | 89.3 | 92.1 | 89.0 | 90.1 |
| | | a | -0.3 | -0.1 | -0.1 | -0.4 | 0.2 | 0.7 | 0.1 |
| | | b | 3.9 | 3.4 | 4.6 | 2.4 | 1.5 | 5.4 | 3.1 |
| | | W | 81.4 | 83.9 | 80.7 | 86.3 | 89.8 | 85.1 | 87.2 |

[Table 6]

| | Evaluation item | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| HAp powder | Bulk density (g/mL) | | 0.4 | 0.5 | 0.8 | 0.5 | 0.4 | 1.2 | 0.6 |
| | Average particle size ($D_{50}$) (μm) | | 92 | 97 | 90 | 43 | 30 | 63 | 13 |
| | Particle size distribution | $D_{10}$ | 57 | 63 | 55 | 31 | 17 | 46 | 6 |
| | | $D_{50}$ | 92 | 97 | 90 | 43 | 30 | 63 | 13 |
| | | $D_{90}$ | 148 | 154 | 147 | 62 | 47 | 94 | 36 |
| | Mode diameter at pore size of 2000 nm or less (mercury porosimetry) (nm) | | 140 | 231 | 620 | 257 | 140 | 337 | 4 |
| | Mode diameter at pore size of 2000 nm or more (mercury porosimetry) (nm) | | 33390 | 90220 | 39320 | 14360 | 8239 | 22050 | 4823 |
| | Pore volume at pore size of 2000 nm or less (mercury porosimetry) (cc/g) | | 0.70 | 0.21 | 0.25 | 0.50 | 0.83 | 0.09 | 0.08 |
| | Pore volume at pore size of 2000 nm or more (mercury porosimetry) (cc/g) | | 0.94 | 0.56 | 0.49 | 0.78 | 0.92 | 0.32 | 0.66 |
| | BET specific surface area ($m^2$/g) | | 19.5 | 4.9 | 1.9 | 4.7 | 15.4 | 0.88 | 1.0 |
| | Average pore size (gas adsorption method) (nm) | | 3.3 | 11.0 | 11.3 | 16.3 | 17.5 | 11.1 | 16.7 |
| | Pore volume (gas adsorption method) (cc/g) | | 0.16 | 0.013 | 0.005 | 0.019 | 0.067 | 0.002 | 0.004 |
| | Crystallinity (%) | | 87 | 97 | 98 | 97 | 89 | 100 | 99 |
| | Angle of repose (°) | | 35.9 | 42.3 | 32.1 | 40.0 | 52.6 | 37.0 | 61.0 |
| | Particle hardness (gf/$mm^2$) | | 75 | 64 | 1295 | Undetectable | Undetectable | 4453 | 313 |
| HAp film | Material of substrate | | PEEK | PEEK | PEEK | PEEK | PEEK | PEEK | PEEK |
| | Cross-sectional hardness (HV0. 3) | | No film was formed | No film was formed | No film was formed | No film was formed | 141 | No film was formed | No film was formed |
| | Abrasion amount (mg) | | | | | | 86 | | |
| | Film thickness (μm) | | | | | | 130 | | |
| | Surface roughness (Ra, μm) | | | | | | 5.5 | | |
| | Crystallinity (%) | | | | | | 72 | | |
| | Impurity phase | α-TCP | | | | | 1.5 | | |
| | | β-TCP | | | | | 1.1 | | |
| | | TTCP | | | | | 0.5 | | |
| | | CaO | | | | | 0.3 | | |
| | Ca/P ratio | | | | | | 1.67 | | |
| | Color difference | L | | | | | 93.7 | | |
| | | a | | | | | 0.0 | | |
| | | b | | | | | 0.4 | | |
| | | W | | | | | 91.9 | | |

## Claims

1. A plasma spraying material comprising a hydroxyapatite powder having an average particle size ($D_{50}$) of 15 to 40 μm and, as measured by mercury porosimetry, a pore volume of 0.01 to 0.30 cc/g at a pore size of 2000 nm or less.

2. The plasma spraying material according to claim 1, wherein the pore volume is 0.01 to 0.25 cc/g.

3. The plasma spraying material according to claim 1 or 2, wherein the average particle size ($D_{50}$) is 20 to 40 μm.

4. The plasma spraying material according to any one of claims 1 to 3, wherein the hydroxyapatite powder has a BET specific surface area of less than 5 m$^2$/g.

5. The plasma spraying material according to any one of claims 1 to 4, wherein the hydroxyapatite powder has a pore volume of 0.20 to 0.80 cc/g at a pore size of 2000 nm or more as measured by mercury porosimetry.

6. The plasma spraying material according to any one of claims 1 to 5, the plasma spraying material to be used for plasma spraying in which a gas consisting of one or more kinds of monatomic molecules as a working gas.

7. The plasma spraying material according to any one of claims 1 to 6, the plasma spraying material to be used for film formation on a substrate.

8. The plasma spraying material according to claim 7, wherein a material of the substrate is a resin, a metal, or a ceramic.

9. The plasma spraying material according to claim 7 or 8, wherein the material of the substrate is a polyether ether ketone.

10. The plasma spraying material according to claim 7 or 8, wherein the material of the substrate is a titanium alloy.

11. The plasma spraying material according to any one of claims 7 to 10, wherein the substrate is an implant.

12. A method for forming a hydroxyapatite film, the method comprising plasma-spraying the plasma spraying material according to any one of claims 1 to 11 to form a hydroxyapatite film on a substrate.

13. The method for forming a hydroxyapatite film according to claim 12, wherein a material of the substrate is a resin, a metal, or a ceramic.

14. The method for forming a hydroxyapatite film according to claim 12 or 13, wherein the material of the substrate is a polyether ether ketone.

15. The method for forming a hydroxyapatite film according to claim 12 or 13, wherein the material of the substrate is a titanium alloy.

16. The method for forming a hydroxyapatite film according to any one of claims 12 to 15, wherein the substrate is an implant.

17. Use of a hydroxyapatite powder as a plasma spraying material, the hydroxyapatite powder having an average particle size ($D_{50}$) of 15 to 40 $\mu$m and, as measured by mercury porosimetry, a pore volume of 0.01 to 0.30 cc/g at a pore size of 2000 nm or less.

**Patentansprüche**

1. Plasmasprühmaterial, umfassend ein Hydroxyapatitpulver, das eine mittlere Teilchengröße ($D_{50}$) von 15 bis 40 $\mu$m und, wie durch Quecksilberporosimetrie gemessen, ein Porenvolumen von 0,01 bis 0,30 cm$^3$/g bei einer Porengröße von 2.000 nm oder weniger aufweist.

2. Plasmasprühmaterial nach Anspruch 1, wobei das Porenvolumen 0,01 bis 0,25 cm$^3$/g beträgt.

3. Plasmasprühmaterial nach Anspruch 1 oder 2, wobei die mittlere Teilchengröße ($D_{50}$) 20 bis 40 $\mu$m beträgt.

4. Plasmasprühmaterial nach einem der Ansprüche 1 bis 3, wobei das Hydroxyapatitpulver eine spezifische BET-Oberfläche von weniger als 5 m$^2$/g aufweist.

5. Plasmasprühmaterial nach einem der Ansprüche 1 bis 4, wobei das Hydroxyapatitpulver ein Porenvolumen von 0,20 bis 0,80 cm$^3$/g bei einer Porengröße von 2.000 nm oder mehr aufweist, wie durch Quecksilberporosimetrie gemessen.

6. Plasmasprühmaterial nach einem der Ansprüche 1 bis 5, wobei das Plasmasprühmaterial zum Plasmasprühen zu verwenden ist, in dem ein Gas aus einer oder mehreren Arten von einatomigen Molekülen als Arbeitsgas besteht.

7. Plasmasprühmaterial nach einem der Ansprüche 1 bis 6, wobei das Plasmasprühmaterial zur Folienbildung auf einem Substrat zu verwenden ist.

8. Plasmasprühmaterial nach Anspruch 7, wobei ein Material des Substrats ein Harz, ein Metall oder Keramik ist.

9. Plasmasprühmaterial nach Anspruch 7 oder 8, wobei das Material des Substrats ein Polyetheretherketon ist.

10. Plasmasprühmaterial nach Anspruch 7 oder 8, wobei das Material des Substrats eine Titanlegierung ist.

11. Plasmasprühmaterial nach einem der Ansprüche 7 bis 10, wobei das Substrat ein Implantat ist.

12. Verfahren zum Bilden einer Hydroxyapatitfolie, wobei das Verfahren das Plasmasprühen eines Plasmasprühmaterials nach einem der Ansprüche 1 bis 11 zum Bilden einer Hydroxyapatitfolie auf einem Substrat umfasst.

13. Verfahren zum Bilden einer Hydroxyapatitfolie nach Anspruch 12, wobei ein Material des Substrats ein Harz, ein Metall oder Keramik ist.

14. Verfahren zum Bilden einer Hydroxyapatitfolie nach Anspruch 12 oder 13, wobei das Material des Substrats ein Polyetheretherketon ist.

15. Verfahren zum Bilden einer Hydroxyapatitfolie nach Anspruch 12 oder 13, wobei das Material des Substrats eine Titanlegierung ist.

16. Verfahren zum Bilden einer Hydroxyapatitfolie nach einem der Ansprüche 12 bis 15, wobei das Substrat ein Implantat ist.

17. Verwendung eines Hydroxyapatitpulvers als Plasmasprühmaterial, wobei das Hydroxyapatitpulver eine mittlere Teilchengröße ($D_{50}$) von 15 bis 40 $\mu$m und, wie durch Quecksilberporosimetrie gemessen, ein Porenvolumen von 0,01 bis 0,30 cm$^3$/g bei einer Porengröße von 2.000 nm oder weniger aufweist.

**Revendications**

1. Matériau de projection au plasma comprenant une poudre d'hydroxyapatite présentant une taille de particule moyenne ($D_{50}$) de 15 à 40 $\mu$m et, tel que mesuré par porosimétrie au mercure, un volume de pores de 0,01 à 0,30 cc/g à une taille de pore de 2 000 nm ou moins.

2. Matériau de projection au plasma selon la revendication 1, dans lequel le volume de pores est de 0,01 à 0,25 cc/g.

3. Matériau de projection au plasma selon la revendication 1 ou 2, dans lequel la taille de particule moyenne ($D_{50}$) est de 20 à 40 $\mu$m.

4. Matériau de projection au plasma selon l'une quelconque des revendications 1 à 3, dans lequel la poudre d'hydroxyapatite présente une surface spécifique BET inférieure à 5 m$^2$/g.

5. Matériau de projection au plasma selon l'une quelconque des revendications 1 à 4, dans lequel la poudre d'hydroxyapatite présente un volume de pores de 0,20 à 0,80 cc/g à une taille de pore de 2 000 nm ou plus telle que mesurée par porosimétrie au mercure.

6. Matériau de projection au plasma selon l'une quelconque des revendications 1 à 5, le matériau de projection au plasma devant être utilisé pour une projection au plasma dans laquelle un gaz consiste en un ou plusieurs types de molécules monoatomiques en tant que gaz de travail.

7. Matériau de projection au plasma selon l'une quelconque des revendications 1 à 6, le matériau de projection au plasma devant être utilisé pour la formation d'un film sur un substrat.

8. Matériau de projection au plasma selon la revendication 7, dans lequel un matériau du substrat est une résine, un métal ou une céramique.

9. Matériau de projection au plasma selon la revendication 7 ou 8, dans lequel le matériau du substrat est une polyétheréthercétone.

10. Matériau de projection au plasma selon la revendication 7 ou 8, dans lequel le matériau du substrat est un alliage de titane.

11. Matériau de projection au plasma selon l'une quelconque des revendications 7 à 10, dans lequel le substrat est un implant.

12. Procédé de formation d'un film d'hydroxyapatite, le procédé comprenant la projection au plasma du matériau de projection au plasma selon l'une quelconque des revendications 1 à 11 pour former un film d'hydroxyapatite sur un substrat.

13. Procédé de formation d'un film d'hydroxyapatite selon la revendication 12, dans lequel un matériau du substrat est une résine, un métal ou une céramique.

14. Procédé de formation d'un film d'hydroxyapatite selon la revendication 12 ou 13, dans lequel le matériau du substrat est une polyétheréthercétone.

15. Procédé de formation d'un film d'hydroxyapatite selon la revendication 12 ou 13, dans lequel le matériau du substrat est un alliage de titane.

16. Procédé de formation d'un film d'hydroxyapatite selon l'une quelconque des revendications 12 à 15, dans lequel le substrat est un implant.

17. Utilisation d'une poudre d'hydroxyapatite comme matériau de projection au plasma, la poudre d'hydroxyapatite présentant une taille de particule moyenne ($D_{50}$) de 15 à 40 $\mu$m et, tel que mesuré par porosimétrie au mercure, un volume de pores de 0,01 à 0,30 cc/g à une taille de pore de 2 000 nm ou moins.

FIG. 1-1

| | Magnification: × 500 | Magnification: × 10000 |
| --- | --- | --- |
| Example 1 | | |
| Example 2 | | |
| Example 3 | | |
| Example 4 | | |

FIG. 1-2

| | Magnification: × 500 | Magnification: × 10000 |
|---|---|---|
| Example 5 | | |
| Example 6 | | |
| Comparative Example 1 | | |

FIG. 1-3

| | Magnification: × 500 | Magnification: × 10000 |
|---|---|---|
| Comparative Example 2 | | |
| Comparative Example 3 | | |
| Comparative Example 4 | | |
| Comparative Example 5 | | |
| Comparative Example 6 | | |
| Comparative Example 7 | | |

FIG. 2-1

| | Magnification: × 500 | Magnification: × 1000 |
|---|---|---|
| Example 1 (PEEK) | | |
| Example 1 (titanium alloy) | | |
| Example 2 | | |
| Example 3 | | |
| Example 4 | | |

FIG. 2-2

| | Magnification: × 500 | Magnification: × 1000 |
|---|---|---|
| Example 5 | | |
| Example 6 | | |
| Comparative Example 5 | | |

FIG. 3

EP 4 190 936 B1

FIG. 4

Example 4

Example 3

Example 4

Example 3

dV/dlog(D) [ml/g]

Diameter [nm]

FIG. 5

FIG. 6

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4146762 A

**Non-patent literature cited in the description**

- **L.BARILLAS et al.** Hydroxyapatite Coatings on Polymers using a Custom Low-Energy Plasma Spray System. *IEEE Transactions on Plasma Science*, July 2018, vol. 46 (7)